(19) Europäisches Patentamt
European Patent Office
Office européen des brevets

(11) EP 3 663 345 B1

(12) **EUROPEAN PATENT SPECIFICATION**

(45) Date of publication and mention of the grant of the patent:
**02.02.2022 Bulletin 2022/05**

(21) Application number: **18886528.1**

(22) Date of filing: **04.12.2018**

(51) International Patent Classification (IPC):
***C08K 5/00*** *(2006.01)* ***C08K 5/103*** *(2006.01)*
***C08L 101/00*** *(2006.01)* ***C08K 5/12*** *(2006.01)*
***C08L 27/06*** *(2006.01)*

(52) Cooperative Patent Classification (CPC):
(C-Sets available)
**C08K 5/103; C08K 5/12; C08L 101/00;**
C08K 5/0016; C08K 2201/014 (Cont.)

(86) International application number:
**PCT/KR2018/015245**

(87) International publication number:
**WO 2019/112292 (13.06.2019 Gazette 2019/24)**

(54) **PLASTICIZER COMPOSITION AND RESIN COMPOSITION COMPRISING SAME**

WEICHMACHERZUSAMMENSETZUNG UND HARZZUSAMMENSETZUNG DAMIT

COMPOSITION DE PLASTIFIANT ET COMPOSITION DE RÉSINE LA COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2017 KR 20170165273**

(43) Date of publication of application:
**10.06.2020 Bulletin 2020/24**

(73) Proprietor: **LG Chem, Ltd.**
**Seoul 07336 (KR)**

(72) Inventors:
- **KIM, Hyun Kyu**
  **Daejeon 34122 (KR)**
- **MOON, Jeong Ju**
  **Daejeon 34122 (KR)**
- **CHO, Yun Ki**
  **Daejeon 34122 (KR)**
- **KIM, Joo Ho**
  **Daejeon 34122 (KR)**
- **JEONG, Seok Ho**
  **Daejeon 34122 (KR)**

(74) Representative: **Plasseraud IP**
**66, rue de la Chaussée d'Antin**
**75440 Paris Cedex 09 (FR)**

(56) References cited:
**EP-A1- 2 851 393**
**KR-A- 20110 133 608**
**KR-A- 20140 132 681**
**KR-A- 20170 130 292**
**US-A- 4 426 477**
**US-A1- 2014 315 021**
**US-A1- 2017 101 524**

- **LAMBERTINI, F. et al.: "Multiresidual LC-MS Analysis of Plasticizers Used in PVC Gaskets of Lids and Assessment of Their Migration into Food Sauces", Journal of Mass Spectrometry, vol. 51, no. 9, 2016, pages 805-813, XP55614451,**
- **SIPES, N. S. et al.: "Profiling 976 ToxCast Chemicals across 331 Enzymatic and Receptor Signaling Assays", Chemical Research in Toxicology, vol. 26, no. 6, 2013, pages 878-895, XP055295960, DOI: doi:10.1021/tx400021f**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08K 5/103, C08L 27/06;**
**C08K 5/12, C08L 27/06**

**Description**

## TECHNICAL FIELD

**[Technical Field]**

The present invention relates to a plasticizer composition and a resin composition including the same.

## BACKGROUND ART

Generally, plasticizers form corresponding esters by the reaction of alcohols with polycarboxylic acids such as phthalic acid and adipic acid. In addition, considering the internal and external regulations on harmful phthalate-based plasticizers to the human body, studies are continuing on plasticizer compositions which may replace phthalate-based plasticizers such as terephthalate-based, adipate-based and other polymer-based plasticizers.

Generally, plasticizers are used as raw materials of diverse products including cables, pipes, flooring materials, wall papers, sheets, artificial leathers, tarpaulins, tapes and food wrapping materials by imparting various processing properties by appropriately adding diverse additives such as a filler, a stabilizer, a pigment, and an anti-fogging agent with a resin such as polyvinyl chloride (PVC) by processing methods including extrusion molding, injection molding and calendaring. KR 2017 0130292 discloses the terephthalate-based material comprising dibutyl terephthalate, butyl(2-ethylhexyl) terephthalate and di(2-ethylhexyl) terephthalate blended with diethylene glycol dibenzoate.

Recently, according to the plasticizer market situation, due to environmental issues on phthalate plasticizers, the development of eco-friendly plasticizers is competitively conducted in the art, and in this course, the development of plasticizer composition products, including vegetable bio products using terephthalate-based, isophthalate-based, adipate-based and vegetable oil raw materials is being conducted. Accordingly, studies on technique for developing better products than commonly used products in the market or novel composition products including one or more thereof to optimally apply as plasticizers for vinyl chloride-based resins, are required.

## DISCLOSURE OF THE INVENTION

## TECHNICAL PROBLEM

The present invention provides a plasticizer which may be applied in a plasticizer composition, which may improve physical properties such as tensile strength, elongation rate and modulus, and may keep the properties of a terephthalate-based material which has excellent transmittance, transparency and migration loss properties, and a resin composition including the same.

## TECHNICAL SOLUTION

To solve the tasks, there is provided in an embodiment of the present invention, a plasticizer composition including a terephthalate-based material including dibutyl terephthalate, butyl(2-ethylhexyl) terephthalate and di(2-ethylhexyl) terephthalate; and a glyceride-based material including at least one compound represented by the following Formula 1:

[Formula 1]

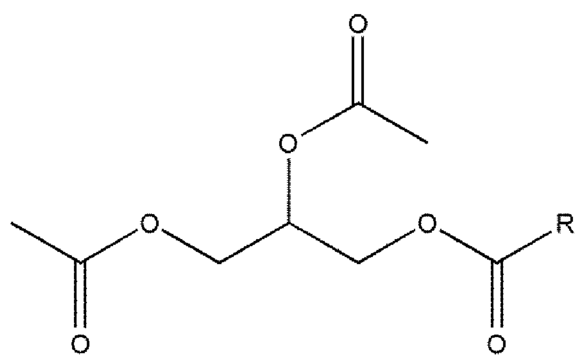

in Formula 1,
R is a linear or branched alkyl group of 8 to 20 carbon atoms.

In order to solve the tasks, there is provided in an embodiment of the present invention, a resin composition including 100 parts by weight of a resin; and 5 to 150 parts by weight of the plasticizer composition.

## ADVANTAGEOUS EFFECTS

The plasticizer composition according to an embodiment of the present invention may serve improved physical properties such as tensile strength, elongation rate and modulus, and may keep the properties of a terephthalate-based material which has excellent transmittance, transparency and migration loss properties.

## MODE FOR CARRYING OUT THE INVENTION

Hereinafter, the present invention will be explained in detail to assist the understanding of the present invention.

It will be understood that terms or words used in the present disclosure and claims should not be interpreted as having a meaning that is defined in common or in dictionaries, however should be interpreted in consistent with the technical scope of the present invention based on the principle that inventors may appropriately define the concept of the terms to explain the invention at his best method.

The term "butyl" used in the description may mean a commonly called n-butyl, and may mean "isobutyl". Hereinafter, the term butyl is not limited to n-butyl but may be used as a term referring to both n-butyl and isobutyl.

### Plasticizer composition

According to an embodiment of the present invention, there is provided a mixture plasticizer composition including three kinds of terephthalate-based materials and a glyceride-based material. Particularly, the terephthalate-based material is characterized in including dibutyl terephthalate, butyl(2-ethylhexyl) terephthalate and di(2-ethylhexyl) terephthalate, and the glyceride-based material is characterized in including at least one compound represented by Formula 1.

The three kinds of the terephthalate-based materials have excellent transmittance or transparency and excellent migration loss properties, and may be advantageously applied to products contacting foods or products contacting the human body, but have defects of having relatively inferior mechanical properties and, if applied to a film type, improvement relating to the unwinding of the film is required.

Meanwhile, the glyceride-based material is a typical eco-friendly material and has excellent plasticization efficiency, but has somewhat poor transparency and transmittance and inferior mechanical properties as well, which may act as fatal defects during commercialization.

The plasticizer composition according to an embodiment of the present invention is a plasticization composition which may solve the above-mentioned defects, and uses materials having no environmental issues as a mixture, thereby improving mechanical properties and keeping the properties of a material having excellent migration loss properties and plasticization efficiency.

The weight ratio of the terephthalate-based material and the glyceride-based material included in the plasticizer composition may be 90:10 to 10:90, where the upper limit thereof may be 90:10, 85:15, 80:20, 70:30 or 60:40 and the lower limit thereof may be 10:90, 15:85, 20:80, 30:70 or 40:60. Preferably, the weight ratio may be 90:10 to 20:80, more preferably, 90:10 to 30:70, the most preferably, 90:10 to 50:50.

If such weight ratio is satisfied, specific physical properties may be kept to excellent levels of each compound as described above and the specific physical properties may be further improved.

The terephthalate-based material is a material in which a diester group is bonded to para positions of a benzene ring, where a 2-ethylhexyl group and a butyl group are bonded to the diester group, and is a mixture of compounds in which two butyl groups, a 2-ethylhexyl group and a butyl group, or two 2-ethylhexyl groups are bonded.

The composition of the three compounds may preferably be 0.5 to 50 wt% of the dibutyl terephthalate; 3.0 to 70 wt% of the butyl(2-ethylhexyl) terephthalate; and 0.5 to 85 wt% of the di(2-ethylhexyl) terephthalate, and the weight ratio may be controlled by adjusting the injection amounts of raw materials during performing reaction. Further, more preferably, the composition of the three compounds may be 0.5 wt% to 50 wt%, 10 wt% to 50 wt%, and 35 wt% to 80 wt%.

In addition, the glyceride-based material may include at least one compound represented by the following Formula 1:

[Formula 1]

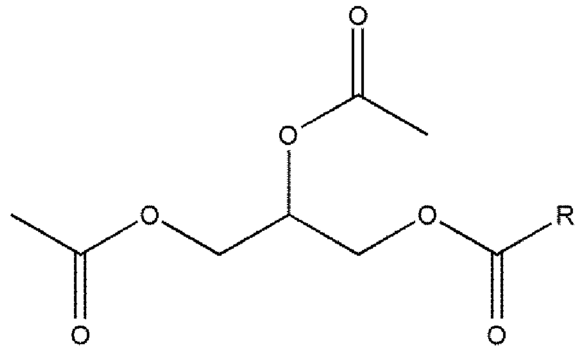

in Formula 1, R is a linear or branched alkyl group of 8 to 20 carbon atoms.

The glyceride-based material may be selected from the compounds in which R is an alkyl group having an even number of carbon atoms among alkyl groups having 8 to 20 carbon atoms, and may preferably be linear. In addition, the glyceride-based material may include at least one compound represented by Formula 1, and in this case, R of each compound may be different from each other. The glyceride-based material may mainly include the compounds having 12 carbon atoms, 14 carbon atoms and 18 carbon atoms.

Generally, in case of mixing two materials, physical properties appeared may show linear change with respect to the physical properties of each material in accordance with the mixing ratio of each material. However, as in the plasticizer composition according to the present invention, if the terephthalate-based material and the glyceride-based material are mixed, excellent properties of each material may be secured and improved mechanical properties when compared with the mechanical properties of two materials, may be achieved.

The plasticizer composition according to another embodiment of the present invention is characterized in including a terephthalate-based material including dibutyl terephthalate, butyl(2-ethylhexyl) terephthalate, di(2-ethylhexyl) terephthalate and terephthalate represented by the following formula 2; and a glyceride-based material including at least one compound represented by the following formula 1, and, based on 100 parts by weight of a mixture weight of the di(2-ethylhexyl) terephthalate and the terephthalate represented by the following Formula 2, the di(2-ethylhexyl) terephthalate is 99.0 parts by weight or more, and the terephthalate represented by the following Formula 2 is less than 1.0 part by weight:

[Formula 2]

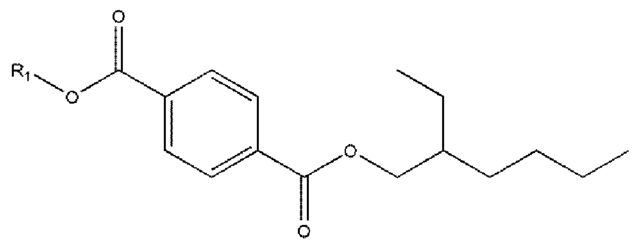

in Formula 2, R1 is a linear or branched alkyl group having 1 to 13 carbon atoms, where R1 is not a 2-ethylhexyl group.

Based on 100 parts by weight of the mixture weight of the di(2-ethylhexyl) terephthalate and the terephthalate represented by Formula 2, the di(2-ethylhexyl) terephthalate may be 99.0 parts by weight or more, and the terephthalate represented by the following Formula 2 may be less than 1.0 part by weight, preferably, 99.2 parts by weight or more and less than 0.8 parts by weight, respectively, more preferably, 99.5 parts by weight or more and less than 0.5 parts by weight, respectively, optimally, 99.9 parts by weight or more and less than 0.1 parts by weight, or 99.95 parts by weight or more and less than 0.05 parts by weight.

Preparation method

A method for preparing the plasticizer composition in the present invention may be a blending method, and the plasticizer composition may be prepared by preparing each of the terephthalate-based material and the glyceride-based

material, and then mixing.

The terephthalate-based material may be prepared by direct esterification of terephthalic acid and two kinds of alcohols, or by the transesterification of di(2-ethylhexyl) terephthalate and butyl alcohol.

In the direct esterification, the alcohol may be 2-ethylhexyl alcohol and butanol, and the mixture alcohol thereof may be applied to the direct esterification.

The direct esterification may be prepared by a step of injecting terephthalic acid to an alcohol, adding a catalyst and reacting under a nitrogen atmosphere; a step of removing unreacted alcohol and neutralizing unreacted acid; and a step of dehydrating by distillation in a reduced pressure and filtering.

In addition, the alcohol may be used in a range of 150 to 500 mol%, 200 to 400 mol%, 200 to 350 mol%, 250 to 400 mol%, or 270 to 330 mol% based on 100 mol% of the terephthalic acid.

Meanwhile, the catalyst of the esterification may be, for example, at least one selected from an acid catalyst such as sulfuric acid, hydrochloric acid, phosphoric acid, nitric acid, paratoluenesulfonic acid, methanesulfonic acid, ethanesulfonic acid, propanesulfonic acid, butanesulfonic acid, and alkyl sulfate, a metal salt such as aluminum lactate, lithium fluoride, potassium chloride, cesium chloride, calcium chloride, iron chloride, and aluminum phosphate, a metal oxide such as heteropoly acids, and an organometal such as natural/synthetic zeolites, cation and anion exchange resins, and tetraalkyl titanate and the polymer thereof. In a particular embodiment, the catalyst may use tetraalkyl titanate.

The amount used of the catalyst may be different according to the kind thereof, and for example, a homogeneous catalyst may be used in an amount of 0.01 to 5 wt%, 0.01 to 3 wt%, 1 to 5 wt% or 2 to 4 wt% based on total 100 wt% of reactants, and a nonhomogeneous catalyst may be used in an amount of 5 to 200 wt%, 5 to 100 wt%, 20 to 200 wt%, or 20 to 150 wt% based on total 100 wt% of reactants.

In this case, the reaction temperature may be within a range of 180 to 280°C, 200 to 250°C, or 210 to 230°C.

In addition, the terephthalate-based material may be prepared by performing transesterification. In case of the transesterification reaction, di(2-ethylhexyl)terephthalate and butyl alcohol may react.

Meanwhile, "transesterification" used in the present invention means the reaction of an alcohol and an ester as shown in Reaction 1 below to interchange R" of the ester with R' of the alcohol as shown in Reaction 1 below.

[Reaction 1]

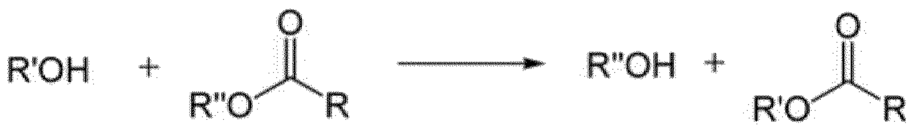

According to an embodiment of the present invention, if the transesterification is carried out, three kinds of ester compositions may be produced according to three cases: a case where the alkoxide of the alcohol attacks the carbon of two ester groups (RCOOR") which are present in the ester-based compound; a case where the alkoxide of the alcohol attacks the carbon of one ester group (RCOOR") which is present in the ester-based compound; and a unreacted case, in which no reaction is performed.

In addition, the transesterification has advantages of not generating waste water problem when compared with the esterification between acid-alcohol, being performed without a catalyst and solving defects occurring when using an acid catalyst.

The composition ratio of the terephthalate-based material which is prepared through the transesterification is the same as described above, and this composition ratio of the mixture may be controlled according to the addition amount of the alcohol.

The amount added of the alcohol may be 0.1 to 89.9 parts by weight, particularly, 3 to 50 parts by weight, more particularly, 5 to 40 parts by weight based on 100 parts by weight of the terephthalate compound.

In regard of the terephthalate, since the mole fraction of the terephthalate compound which participates in the transesterification may increase according to the increase of the amount added of the alcohol, the amounts of two terephthalate compounds which are products in the mixture may increase. Correspondingly, the amount of the terephthalate compound which is present in an unreacted state, tends to decrease.

According to an embodiment of the present invention, the molar ratio of the reactants, terephthalate and alcohol may be, for example, 1:0.005 to 5.0, 1:0.05 to 2.5, or 1:0.1 to 1.0, and within this range, and an ester-based plasticizer composition having high processing efficiency and excellent processability improving effect may be obtained.

The composition ratio may be the ratio of a mixture composition obtained by the esterification, and may be a desired composition ratio by further mixing a specific compound. The mixture composition ratio may be appropriately controlled so as to achieve desired physical properties. However, the mixture composition ratio of the three kinds of the

terephthalate-based materials is not limited to the range. The composition ratio may be changed by additionally injecting any one among the three kinds of the terephthalate, and available mixing composition ratio is the same as described above.

According to an embodiment of the present invention, the transesterification may be performed at 120 to 190°C, preferably, 135 to 180°C, more preferably, 141 to 179°C for 10 minutes to 10 hours, preferably, 30 minutes to 8 hours, more preferably, 1 to 6 hours. Within the temperature and time ranges, a mixture which is a terephthalate-based material having a desired composition ratio may be effectively obtained. In this case, the reaction time may be calculated from a point when the reaction temperature is attained after elevating the temperature of the reactants.

The transesterification may be performed under an acid catalyst or a metal catalyst, and in this case, the effects of decreasing reaction time may be achieved.

The acid catalyst may include, for example, sulfuric acid, methanesulfonic acid or p-toluenesulfonic acid, and the metal catalyst may include, for example, an organometal catalyst, a metal oxide catalyst, a metal salt catalyst, or a metal itself.

The metal component may be, for example, any one selected from the group consisting of tin, titanium and zirconium, or a mixture of two or more thereof.

The direct esterification and the transesterification may be used for preparing the glyceride material described above. That is, particular reaction conditions, molar ratio, etc. may be similar.

The glyceride-based material may be generally prepared using a vegetable oil as a raw material, and may be used together with glycerin, acetic acid (or acetic anhydride) and triacetin materials as supplementary materials.

For example, as a first method, the compound represented by Formula 1 may be prepared by; a direct esterification step for reacting glycerin and acetic acid in the presence of a catalyst to produce triacetin; and a transesterification step for reacting a vegetable oil and the triacetin in the presence of a catalyst. That is, the compound represented by Formula 1 may be prepared via acetylation first and transesterification of triacetin which is obtained by the acetylation of glycerin with vegetable oil.

In addition, as a second method, the compound represented by Formula 1 may be prepared by; an esterification step of vegetable oil and glycerin; and a step of reacting the transesterification product and acetic acid. Different from the first method, the esterification and acetylation in the second method may be performed in a reverse order.

As the vegetable oil, for example, almond oil, avocado oil, castor oil, corn oil, cottonseed oil, olive oil, peanut oil, rice bran oil, safflower oil, sesame oil, soybean oil, sunflower oil, refined palm oil, palm kernel oil, coconut oil, canola oil, etc. may be used.

Particular conditions of the esterification and kinds of the catalyst may not much different from the explanation above. The acid may be replaced with an acid anhydride, and after finishing the reaction, general processes for commercialization including purification may be performed.

The terephthalate-based material and the glyceride-based material thus prepared may be blended by a common method, and the blending method is not specifically limited.

Resin composition

According to another embodiment of the present invention, the plasticizer composition may be included in an amount of 5 to 150 parts by weight, 10 to 100 parts by weight, or 30 to 60 parts by weight and 70 to 130 parts by weight according to the use applied, based on 100 parts by weight of a resin including ethylene vinyl acetate, polyethylene, polyketone, polypropylene, polyvinyl chloride, polystyrene, polyurethane, thermoplastic elastomer, or a mixture thereof.

The resin composition may be processed through various methods such as plastisol processing, extrusion or injection processing, and calendaring processing, and may be applied to cables, car interior materials, films, sheets, tubes, wall papers, toys, flooring materials, wirings or coating materials of optical fibers.

In addition, the resin composition may include products designed for utilizing in a medical or food industry, for example, blood bags, intravenous injection bags, saline bags, intravenous injection tubes, stomach tubes, catheter tubes, drainage tubes, medical gloves, oxygen masks, correction-support apparatuses, artificial skins and food wrapping materials (for example, wrapping materials for various beverages, meats and frozen vegetables).

Preferably, the resin composition may be applied to an eco-friendly resin for wrapping foods or medical resins and may be evaluated to have excellent functionalities including transparency and color so as to be applied to the resins, and may show excellent adhesion and similar or better basic mechanical properties such as plasticization efficiency and volatile loss as the conventional plasticizer.

To the resin composition, a stabilizer, an anti-fogging agent, etc. may be additionally added, and other additives may be further added.

**Examples**

Hereinafter, embodiments will be explained in detail to particularly explain the present invention. The present invention may, however, be embodied in different forms and should not be construed as limited to the embodiments set forth herein. Rather, these embodiments are provided so that this disclosure will be thorough and complete, and will fully convey the scope of the inventive concept to those skilled in the art.

**Preparation Example 1: Preparation of terephthalate-based material**

To a reactor equipped with a stirrer, a condenser and a decanter, 2000 g of di(2-ethylhexyl) terephthalate (LG Chem,) and 340 g of n-butanol (17 parts by weight based on 100 parts by weight of DEHTP) were injected, and transesterification was carried out under a nitrogen atmosphere at a reaction temperature of 160°C for 2 hours to obtain a composition including dibutyl terephthalate (DBTP), butyl(2-ethylhexyl) terephthalate (BEHTP) and di(2-ethylhexyl) terephthalate (DEHTP) in amounts of 4.0 wt%, 35.0 wt% and 61.0 wt%, respectively.

The reaction product was distilled to remove butanol and 2-ethylhexyl alcohol to finally prepare a mixture composition.

**Preparation Example 2: Preparation of glyceride-based material**

To the same apparatus as in Preparation Example 1, 1000 g of coconut oil and 300 g of glycerin were injected, and transesterification was performed at a reaction temperature of 100°C for 4 hours and the reaction product was purified to obtain monoglyceride. Then, acetylation was performed at 120°C using an excessive amount of acetic anhydride, and extraction and purification processes were performed to obtain 1510 g of a final product.

The materials prepared in Preparation Examples 1 and 2 were mixed to prepare plasticizer compositions of the examples, and the particulars are summarized in Table 1 below. The evaluation of the physical properties of the plasticizer compositions was performed according to the test items below.

[Table 1]

| | Material of Preparation Example 1 | Material of Preparation Example 2 |
|---|---|---|
| Example 1 | 90 | 10 |
| Example 2 | 70 | 30 |
| Example 3 | 50 | 50 |
| Example 4 | 30 | 70 |
| Example 5 | 10 | 90 |
| Comparative Example 1 | 100 | 0 |
| Comparative Example 2 | 0 | 100 |

<Test items>

Hardness measurement

Shore (Shore A and D) hardness at 25°C, 3T 10s was measured according to ASTM D2240. The lower the value was, the better.

Tensile strength measurement

By ASTM D638 method, a specimen was drawn in a crosshead speed of 100 mm/min (0.25 T) using a test apparatus of U.T.M (manufacturer: Instron, model name: 4466), and a point where the specimen was cut was measured. The tensile strength was measured in a TD direction and a MD direction and was calculated as follows. The higher the value was, the better.

$$\text{Tensile strength (kgf/mm}^2\text{) = load value (kgf)/thickness (mm) x width (mm)}$$

Elongation rate measurement

By ASTM D638 method, a specimen was drawn in a crosshead speed of 100 mm/min (0.25 T) using a test apparatus of U.T.M, and a point where the specimen was cut was measured. The elongation rate was measured in a TD direction and a MD direction and was calculated as follows. The higher the value was, the better.

$$\text{Elongation rate (\%) = [length after elongation/initial length] x 100}$$

Migration loss measurement

According to KSM-3156, a specimen (1 T) with a thickness of 2 mm or more was obtained, PS plates were attached onto both sides of the specimen and a load of 98066.5 $N/m^2$ (1 $kgf/cm^2$) was applied. The specimen was stood in a hot air circulation type oven (80°C) for 72 hours and then taken out and cooled at room temperature for 4 hours. Then, the PS plates attached onto both sides of the specimen were removed, the weights before and after standing in the oven were measured, and the migration loss was calculated as follows. The lower the value was, the better.

$$\text{Migration loss (\%) = [(initial weight of specimen at room temperature - weight of specimen after standing in oven)/initial weight of specimen at room temperature] x 100}$$

Volatile loss measurement

The specimen manufactured was processed at 80°C for 72 hours, the weight of the specimen was measured, and calculation was conducted as follows. The lower the value was, the better.

$$\text{Volatile loss (\%) = [(initial weight of specimen - weight of specimen after processing)/initial weight of specimen] x 100}$$

100% modulus measurement

By ASTM D638 method, a specimen was drawn in a crosshead speed of 100 mm/min (0.25 T) using a test apparatus of U.T.M, and the elongation stress (100% modulus) when elongated by 100% was measured in a TD direction and a MD direction. The lower the value was, the better.

Haze and transparency measurement

By using NDH 7000 Haze meter, haze and transparency were measured. The lower the haze value was, the better, and the higher the transparency value was, the better.

**Experimental Example 1: Evaluation of physical properties of resin specimen**

A specimen was manufactured using each of the mixture plasticizer compositions of the Examples and the Comparative Examples as listed in Table 1.

Each specimen was manufactured referring to ASTM D638. With respect to 100 parts by weight of a polyvinyl chloride resin (PVC (LS100), 40 parts by weight of each plasticizer composition prepared in the Examples and the Comparative Examples, 10 parts by weight of epoxidized soybean oil (ESO), 1.5 parts by weight of LTX-630P as a stabilizer, and 2 parts by weight of Almax-9280 as an anti-fogging agent were blended and mixed in 700 rpm at 98°C. by using a roll mill, working was conducted at 160°C for 4 minutes and processing using a press was conducted at 180°C for 2.5 minutes (low pressure) and 2 minutes (high pressure) to manufacture a specimen.

Using each specimen, the test items were evaluated and the results are listed in Table 2 below.

[Table 2]

| | | Exampl e 1 | Exampl e 2 | Exampl e 3 | Exampl e 4 | Exampl e 5 | Comparative Example 1 | Comparative Example 2 |
|---|---|---|---|---|---|---|---|---|
| Hardness | Shore A | 82.3 | 82.3 | 82.2 | 82.0 | 81.8 | 83.8 | 81.8 |
| | Shore D | 36.8 | 36.7 | 36.7 | 36.5 | 36.4 | 37.8 | 36.4 |
| Tensile strength ($N/m^2$ ) | TD | 204174 45.3 (208.2 $kgf/cm^2$) | 202703 45.55 (205.9 $kgf/cm^2$) | 202703 45,6 (206.7 $kgf/cm^2$) | 202016 99.0 (206.0 $kgf/cm^2$) | 200545 99.3 (204.5 $kgf/cm^2$) | 20593965.0 (210.0 $kgf/cm^2$) | 19221034.0 (196.0 $kgf/cm^2$) |
| | MD | 200226 739951 2,5 (227.0 $kgf/cm^2$) | 198014 821114 1,3 (225.6 $kgf/cm^2$) | 198784 184187 9,1 (225.1 $kgf/cm^2$) | 198110 991498 3,5 (223.9 $kgf/cm^2$) | 196668 435735 0,1 (223. $kgf/cm^2$) 7 | 20195780686 72,5 (228.7 $kgf/cm^2$) | 1884939530761 , 0 (208.3 $kgf/cm^2$) |
| Elongation rate (%) | TD | 292.5 | 292.7 | 291.3 | 292.0 | 293.0 | 293.2 | 293.4 |
| | MD | 305.0 | 306.6 | 307.5 | 304.0 | 306.7 | 294.0 | 305.4 |
| 100% Modulus | TD | 91.5 | 91.3 | 91.4 | 91.2 | 90.9 | 94.0 | 90.8 |
| | MD | 96.2 | 94.9 | 94.6 | 94.8 | 94.2 | 101.1 | 94.0 |
| Migration loss (%) | | 1.38 | 1.42 | 1.66 | 1.74 | 2.21 | 1.33 | 4.10 |
| Volatile loss (%) | | 2.01 | 2.13 | 2.45 | 2.50 | 2.68 | 1.90 | 3.25 |
| Haze (%) | | 3.20 | 3.33 | 3.37 | 3.60 | 3.80 | 3.18 | 5.88 |
| Transparency (%) | | 91.2 | 90.5 | 90.4 | 89.5 | 88.7 | 91.1 | 85.9 |

Referring to Table 2, the hardness values of Examples 1 to 4 were evaluated to be an equivalent degree to the excellent hardness value of the glyceride-based material of Comparative Example 2 and thus, the plasticization efficiency was found to be taken from better side properties. When comparing mechanical properties including tensile strength, elongation rate and modulus values of the Examples with Comparative Examples 1 and 2, the values were equivalent degrees as the materials having excellent values. In addition, the migration loss and the volatile loss of the Examples were equivalent to Comparative Example 1 which had a lower value, and it was found that excellent physical properties were taken. The haze value and transparency were also found to be equivalent degrees to the values of a better one.

Particularly, with respect to the elongation rate properties, the elongation rates in a TD direction of the terephthalate-based material and the glyceride-based material were similar but the elongation rate in a MD direction was better for the glyceride-based material of Comparative Example 2. If two materials were mixed, the elongation rates in both the TD direction and the MD direction were excellent, and particularly, the elongation rate in the MD direction showed even further improved value.

Through this, it may be found that if a terephthalate-based material and a glyceride-based material are mixed, excellent physical properties of each material may be kept to the same or better degrees and mechanical properties may show even further improved values. Accordingly, the plasticizer composition according to the present invention has excellent plasticization efficiency and improved mechanical properties, and may provide a resin which may keep excellent degree of volatile loss, migration loss, haze and transparency.

That is, as the effects anticipated from the mixing of two materials, linear change of the physical properties of each material was not shown, but excellent physical properties of each material were kept to the same or better degrees and some properties were even further improved.

## Claims

1. A plasticizer composition, comprising:

    a terephthalate-based material comprising dibutyl terephthalate, butyl(2-ethylhexyl) terephthalate and di(2-ethylhexyl) terephthalate; and
    a glyceride-based material comprising at least one compound represented by the following Formula 1:

    [Formula 1]

    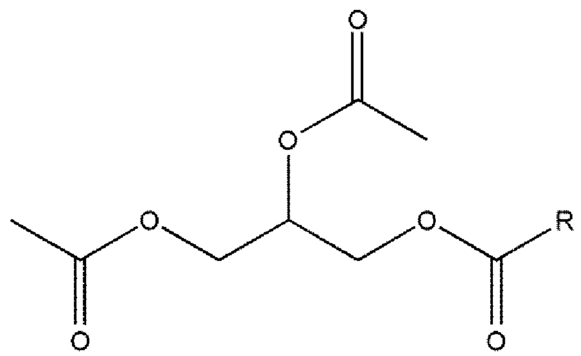

    in Formula 1,
    R is a linear or branched alkyl group of 8 to 20 carbon atoms.

2. The plasticizer composition according to claim 1, wherein a weight ratio of the terephthalate-based material and the glyceride-based material is 90:10 to 10:90.

3. The plasticizer composition according to claim 1, wherein the weight ratio of the terephthalate-based material and the glyceride-based material is 90:10 to 30:70.

4. The plasticizer composition according to claim 1, wherein the terephthalate-based material comprises 0.5 to 30 wt% of the dibutyl terephthalate; 10 to 50 wt% of the butyl(2-ethylhexyl) terephthalate; and 40 to 89 wt% of the di(2-ethylhexyl) terephthalate.

**5.** A plasticizer composition according to claim 1, comprising:

a terephthalate-based material comprising dibutyl terephthalate, butyl(2-ethylhexyl) terephthalate, di(2-ethylhexyl) terephthalate, and terephthalate represented by the following Formula 2; and
a glyceride-based material comprising at least one compound represented by the following formula 1,
wherein, based on 100 parts by weight of a mixture weight of the di(2-ethylhexyl) terephthalate and the terephthalate represented by the following formula 2, the di(2-ethylhexyl) terephthalate is 99.0 parts by weight or more, and the terephthalate represented by the following Formula 2 is less than 1.0 part by weight:

[Formula 1]

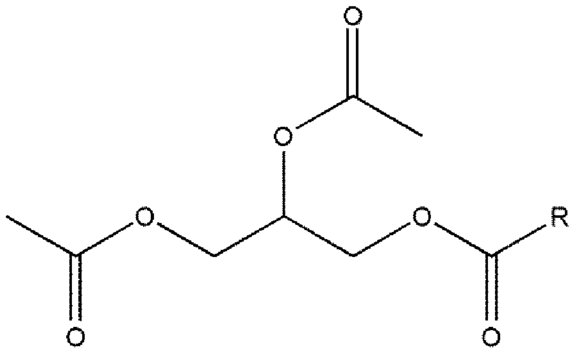

in Formula 1,
R is a linear or branched alkyl group of 8 to 20 carbon atoms,

[Formula 2]

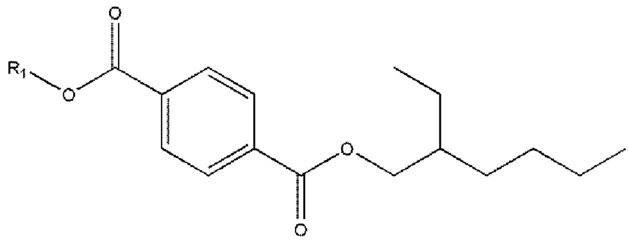

in Formula 2,
$R_1$ is a linear or branched alkyl group having 1 to 13 carbon atoms, where $R_1$ is not a 2-ethylhexyl group.

**6.** A resin composition comprising 100 parts by weight of a resin; and 5 to 150 parts by weight of the plasticizer composition according to claim 1 or 5.

**7.** The resin composition according to claim 6, wherein the resin is at least one selected from the group consisting of ethylene vinyl acetate, polyethylene, polypropylene, polyketone, polyvinyl chloride, polystyrene, polyurethane and thermoplastic elastomer.

**8.** The resin composition according to claim 6, wherein the resin composition is a material of at least one product selected from the group consisting of cables, flooring materials, car interior materials, films, sheets, wall papers, and tubes.

**Patentansprüche**

**1.** Weichmacherzusammensetzung, umfassend:

ein Material auf Terephthalatbasis, umfassend Dibutylterephthalat, Butyl(2-ethylhexyl)terephthalat und Di(2-ethylhexyl)terephthalat; und
ein Material auf Glyceridbasis, umfassend mindestens eine Verbindung, dargestellt durch die folgende Formel 1:

[Formel 1]

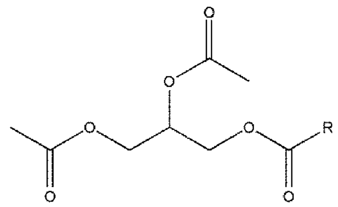

wobei in Formel 1 R eine lineare oder verzweigte Alkylgruppe mit 8 bis 20 Kohlenstoffatomen ist.

**2.** Weichmacherzusammensetzung nach Anspruch 1, wobei ein Gewichtsverhältnis des Materials auf Terephthalatbasis und des Materials auf Glyceridbasis 90:10 bis 10:90 beträgt.

**3.** Weichmacherzusammensetzung nach Anspruch 1, wobei das Gewichtsverhältnis des Materials auf Terephthalatbasis und des Materials auf Glyceridbasis 90:10 bis 30:70 beträgt.

**4.** Weichmacherzusammensetzung nach Anspruch 1, wobei das Material auf Terephthalatbasis 0,5 bis 30 Gew.-% des Dibutylterephthalats; 10 bis 50 Gew.-% des Butyl-(2-ethylhexyl)terephthalats; und 40 bis 89 Gew.-% des Di(2-ethylhexyl)terephthalats umfasst.

**5.** Weichmacherzusammensetzung nach Anspruch 1, umfassend:

ein Material auf Terephthalatbasis, umfassend Dibutylterephthalat, Butyl(2-ethylhexyl)terephthalat, Di(2-ethylhexyl)terephthalat und Terephthalat, dargestellt durch die folgende Formel 2; und
ein Material auf Glyceridbasis, umfassend mindestens eine Verbindung, dargestellt durch die folgende Formel 1, wobei, bezogen auf 100 Gewichtsteile eines Mischungsgewichts des Di(2-ethylhexyl)terephthalats und des Terephthalats, dargestellt durch die folgende Formel 2, das Di(2-ethylhexyl)terephthalat 99,0 Gewichtsteile oder mehr beträgt und das Terephthalat, dargestellt durch die folgende Formel 2, weniger als 1,0 Gewichtsteile beträgt:

[Formel 1]

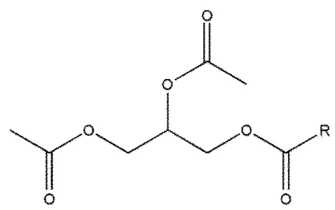

wobei in Formel 1 R eine lineare oder verzweigte Alkylgruppe mit 8 bis 20 Kohlenstoffatomen ist,

[Formel 2]

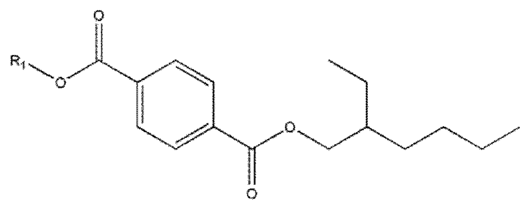

wobei in Formel 2 $R_1$ eine lineare oder verzweigte Alkylgruppe mit 1 bis 13 Kohlenstoffatomen ist, wobei $R_1$ keine 2-Ethylhexylgruppe ist.

6. Harzzusammensetzung, umfassend 100 Gewichtsteile eines Harzes; und 5 bis 150 Gewichtsteile der Weichmacherzusammensetzung nach Anspruch 1 oder 5.

7. Harzzusammensetzung nach Anspruch 6, wobei das Harz mindestens eines ausgewählt aus der Gruppe bestehend aus Ethylenvinylacetat, Polyethylen, Polypropylen, Polyketon, Polyvinylchlorid, Polystyrol, Polyurethan und thermoplastischem Elastomer ist.

8. Harzzusammensetzung nach Anspruch 6, wobei die Harzzusammensetzung ein Material von mindestens einem Produkt ist, ausgewählt aus der Gruppe bestehend aus Kabeln, Bodenbelagsmaterialien, Fahrzeuginnenraummaterialien, Folien, Platten, Tapeten und Schläuchen.

## Revendications

1. Composition de plastifiant, comprenant :

un matériau à base de téréphtalate comprenant du téréphtalate de dibutyle, du téréphtalate de butyle et de (2-éthylhexyle) et du téréphtalate de di(2-éthylhexyle) ; et
un matériau à base de glycéride comprenant au moins un composé représenté par la formule 1 suivante :

[Formule 1]

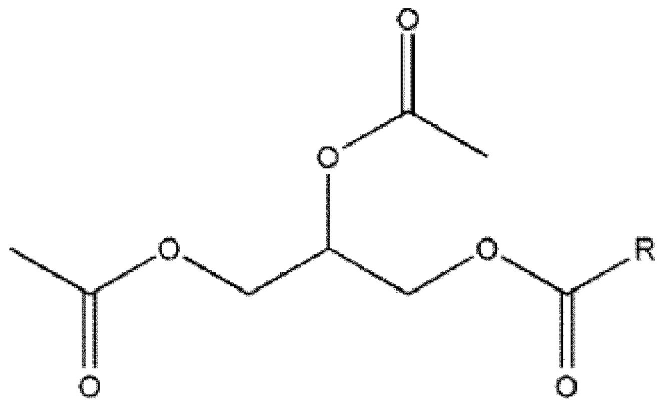

dans la formule 1,
R est un groupe alkyle linéaire ou ramifié de 8 à 20 atomes de carbone.

2. Composition de plastifiant selon la revendication 1, dans laquelle un rapport en poids du matériau à base de téréphtalate et du matériau à base de glycéride est de 90:10 à 10:90.

3. Composition de plastifiant selon la revendication 1, dans laquelle le rapport en poids du matériau à base de téréphtalate et du matériau à base de glycéride est de 90:10 à 30:70.

4. Composition de plastifiant selon la revendication 1, dans laquelle le matériau à base de téréphtalate comprend 0,5 à 30 % en poids du téréphtalate de dibutyle ; 10 à 50 % en poids du téréphtalate de butyle et de (2-éthylhexyle) ; et 40 à 89 % en poids du téréphtalate de di(2-éthylhexyle).

5. Composition de plastifiant selon la revendication 1, comprenant :

un matériau à base de téréphtalate comprenant du téréphtalate de dibutyle, du téréphtalate de butyle et de (2-éthylhexyle), du téréphtalate de di(2-éthylhexyle), et un téréphtalate représenté par la formule 2 suivante ; et
un matériau à base de glycéride comprenant au moins un composé représenté par la formule 1 suivante,
dans laquelle, sur la base de 100 parties en poids d'un mélange en poids du téréphtalate de di(2-éthylhexyle)

et du téréphtalate représenté par la formule 2 suivante, le téréphtalate de di(2-éthylhexyle) est à 99,0 parties en poids ou plus, et le téréphtalate représenté par la formule 2 suivante est à moins de 1,0 partie en poids :

[Formule 1]

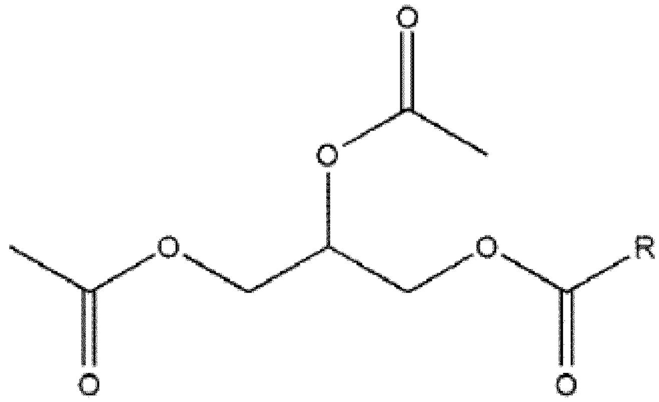

dans la formule 1,
R est un groupe alkyle linéaire ou ramifié de 8 à 20 atomes de carbone.

[Formule 2]

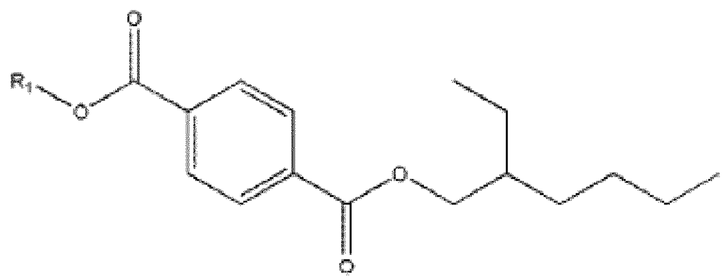

dans la formule 2,
$R_1$ est un groupe alkyle linéaire ou ramifié ayant 1 à 13 atomes de carbone, où $R_1$ n'est pas un groupe 2-éthylhexyle.

**6.** Composition de résine comprenant 100 parties en poids d'une résine ; et 5 à 150 parties en poids de la composition de plastifiant selon la revendication 1 ou 5.

**7.** Composition de résine selon la revendication 6, dans laquelle la résine est au moins une sélectionnée dans le groupe constitué d'un éthylène-acétate de vinyle, d'un polyéthylène, d'un polypropylène, d'une polycétone, d'un polychlorure de vinyle, d'un polystyrène, d'un polyuréthane et d'un élastomère thermoplastique.

**8.** Composition de résine selon la revendication 6, dans laquelle la composition de résine est un matériau d'au moins un produit sélectionné dans le groupe constitué des câbles, des matériaux de revêtement de sol, des matériaux d'intérieur de voiture, des films, des feuilles, des papiers peints, et des tubes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20170130292